# EUROPÄISCHE PATENTANMELDUNG

(11) **EP 4 772 149 A1**
(43) Veröffentlichungstag der Anmeldung: **08.07.2026**
(21) Anmeldenummer: 25211903.7
(22) Anmeldetag: 29.10.2025
(51) Int. Cl.: A61F 9/00, A61F 9/007

(54) **EINSETZHILFE ZUM EINSETZEN EINER SKLERALLINSE**

(71) Anmelder: Appenzeller Kontaktlinsen AG, 9042 Speicher (CH)
(72) Erfinder: BOUHLAL, M'hamed, 9042 SPEICHER (CH)
(74) Vertreter: Eisenführ Speiser

(57) **Zusammenfassung**

Die Erfindung betrifft eine Einsetzhilfe (1) zum Einsetzen von Sklerallinsen (6), mit einem Ständer (2) aufweisend ein proximales Ende (8) und ein distales Ende (10), wobei der Ständer (2) an dem distalen Ende (10) einen Ständerfuss (12) zum Aufstellen auf einer Arbeitsfläche aufweist, und einem Sauger (4) zum Aufnehmen der Sklerallinse (6), wobei der Sauger (6) an dem proximalen Ende (8) des Ständers (2) angeordnet ist. Gemäß der Erfindung weist der Ständer (2) eine passive Lichtleiteinrichtung (20) zum Leiten von Umgebungslicht durch den Ständer (2) bis zum proximalen Ende (8) des Ständers (2) auf, zum Beleuchten der Sklerallinse (6) durch den Sauger (4) hindurch.

## Beschreibung

Die Anmeldung betrifft den Bereich der ophthalmischen Hilfsmittel und bezieht sich auf eine Einsetzhilfe zum Einsetzen von Sklerallinsen, insbesondere auf Vorrichtungen mit Ständer und Sauger zur mechanischen Bereitstellung der Linse sowie auf Lösungen zur optischen Unterstützung der Sicht während des Einsetzens.

Sklerallinsen unterscheiden sich von herkömmlichen Kontaktlinsen dadurch, dass sie mit ihrer Auflagefläche auf der Sklera, also dem weißen Teil des Auges, aufliegen und über der Hornhaut einen Flüssigkeitsspalt bilden. Beim Einsetzen einer Sklerallinse muss dieser Flüssigkeitsspalt vollständig mit Kochsalzlösung oder einer geeigneten Füllflüssigkeit gefüllt sein, um Luftblasen zwischen Hornhaut und Linse zu vermeiden. Das Einsetzen erfordert daher eine präzise Ausrichtung der Linse gegenüber dem Auge sowie ein ruhiges Handling, da jede kleine Bewegung zu Luftblasenbildung oder einer fehlerhaften Positionierung führen kann.

Aus dem Stand der Technik sind verschiedene Einsetzhilfen für Sklerallinsen bekannt. Eine Gruppe von Vorrichtungen stellt die Linse mittels Ständer und Sauger mechanisch bereit, verzichtet jedoch auf eine integrierte Beleuchtung. Bei alltäglichen, nicht idealen Lichtverhältnissen (z. B. Schattenwurf, diffuse Beleuchtung, niedrige Beleuchtungsstärke) bleibt die Sicht auf die Linse, den Flüssigkeitsmeniskus und die Ausrichtung eingeschränkt. Dies kann die Handhabung erschweren und die Fehleranfälligkeit erhöhen.

Demgegenüber existieren Lösungen mit aktiver Beleuchtung, die eine elektrische Energieversorgung (z. B. Batterien oder externe Stromquellen) benötigen. Solche Systeme erhöhen die Bau- und Bedienkomplexität, sind wartungsintensiv (Batteriewechsel/Ladevorgänge) und verursachen zusätzlichen Ressourcenverbrauch. Darüber hinaus führen aktive Komponenten (Elektronik, Schalter, Kontakte) zu potenziellen Ausfallursachen, erschweren die Reinigung und können die Robustheit im feuchtigkeitsnahen Anwendungskontext beeinträchtigen.

So beschreibt beispielsweise das SeeGreen^{®} Scleral Lens Insertion System einen Ständer mit integrierter LED-Lichtquelle, die zur Ausrichtung des Blicks des Anwenders auf einen grünen Lichtpunkt dient. Der Ständer trägt eine Haltevorrichtung für die Linse, sodass der Benutzer beide Hände frei hat, um die Lider zu öffnen und das Auge langsam auf die Linse abzusenken. Ähnliche Konzepte finden sich bei der Vorrichtung i-INSERT der hiesigen Anmelderin, die ebenfalls eine Ständerstruktur mit Beleuchtung vorsieht, sowie beim S5 Inserter, der eine aktive LED-Beleuchtung und eine verstellbare Auflageplattform kombiniert.

Vor diesem Hintergrund besteht Bedarf an einer Einsetzhilfe, die die Sichtbarkeit der Sklerallinse während des Einsetzens verbessert. Insbesondere soll eine Lösung bereitgestellt werden, die in unterschiedlichen Umgebungslichtsituationen zuverlässig nutzbar ist und zugleich eine hohe Alltagstauglichkeit, Reinigbarkeit und Robustheit aufweist.

Die Aufgabe wird durch die in Anspruch 1 definierte Vorrichtung gelöst. Insbesondere wird sie dadurch gelöst, dass ein mechanisch einfacher Ständer mit einem am distalen Ende vorgesehenen Ständerfuß und einem am proximalen Ende angeordneten Sauger mit einer passiven Lichtleiteinrichtung kombiniert wird, die Umgebungslicht bis in den Bereich des Saugers leitet und die Sklerallinse durch den Sauger hindurch beleuchtet. Hierdurch wird die Sichtbarkeit der Linse und ihres Flüssigkeitsmeniskus verbessert, ohne aktive Komponenten oder Energiequellen einzusetzen und bei gleichzeitig geringer Baukomplexität.

Anspruch 1 sieht hierfür folgende funktionale Gliederung vor: ein "Ständer" (tragendes Strukturbauteil, vorzugsweise freistehend, das die räumliche Anordnung der Komponenten gewährleistet), ein "proximales Ende" (dem Anwender und der Linse zugewandter Endbereich des Ständers) und ein "distales Ende" (von der Linse abgewandter Endbereich, zur Abstützung auf einer Fläche) sowie einen "Ständerfuß" (Stand- oder Auflageelement zum stabilen Aufstellen auf einer "Arbeitsfläche", vorzugsweise jede geeignete horizontale oder leicht geneigte Abstellfläche.

Die Einsetzhilfe weist ferner einen "Sauger" (Halteelement zum Aufnehmen der Sklerallinse, z. B. ein elastischer Saugnapf oder cup-förmiger Inserter) auf, der vorzugsweise zumindest bereichsweise lichtdurchlässig ist. Der Sauger kann beispielsweise abschnittsweise oder vollständig aus einem transparenten Kunststoff gebildet sein. Ebenso kann der Sauger einen zentralen Durchlass aufweisen.

Gemäß der Erfindung ist eine passive Lichtleiteinrichtung vorgesehen, die Umgebungslicht (vorhandenes Tages- oder Raumlicht, auch gerichtetes Kunstlicht in der Umgebung) durch den Sauger hindurch bis zum Linsenbereich führt. Hierdurch wird eine einfache, wartungsfreie und nachhaltige Vorrichtung, welche die optische Unterstützung beim Einsetzen von Sklerallinsen verbessert, ohne dass elektronische Komponenten erforderlich sind, bereitgestellt. Durch die Nutzung des vorhandenen Umgebungslichts und dessen gezielte Bündelung wird ein technischer Effekt erzielt, der mit den rein mechanischen Vorrichtungen des Stands der Technik nicht erreichbar war.

In einer bevorzugten Ausführungsform ist am proximalen Ende des Ständers ein napfförmiger Aufnahmekörper vorgesehen, in den der Sauger lösbar eingesetzt wird. Die Lösbarkeit kann durch Presssitz, Schnappverbindung, Bajonett- oder Gewindeanschluss realisiert werden. Der Aufnahmenapf ermöglicht den Austausch unterschiedlicher Saugergeometrien und erleichtert Reinigung/Desinfektion. Ein zentrischer Bodenabschnitt des Aufnahmenapfes kann der definierten Positionierung des Saugers dienen. Es kann aber auch Ausführungsformen geben, in denen der Sauer einstückig und/oder unlösbar mit dem Ständer verbunden ist bzw. an diesem ausgeformt ist.

In einer bevorzugten Weiterbildung weist die Lichtleiteinrichtung ein Lichtleitkabel auf, das vorzugsweise von einer Außenseite des Ständers bis zum proximalen Ende geführt ist. Hierdurch lässt sich Umgebungslicht in den Linsenbereich einkoppeln. Geeignete Lichtleitkabel bzw. Lichtwellenleiter sind z. B. PMMA-/Polycarbonat-Monofasern, Glasfaserbündel oder flexible Lichtleitstäbe mit Totalreflexion. Die Außenseite des Ständers dient als Lichteinkopplungsbereich und kann mit einer optischen Eintrittsfläche (plan, konvex) ausgebildet sein, um den Einfallswinkel des Umgebungslichts zu begünstigen.

Das Lichtleitkabel erstreckt sich bevorzugt bis in den Bodenabschnitt des Aufnahmenapfes. Bevorzugt wird eine direkte, koaxiale Beleuchtung des Bereichs unterhalb der Linse durch den Sauger hindurch erzielt. Diese Führung minimiert Abschattung und Streuverluste und stabilisiert die Ausrichtung zwischen Lichtaustritt und Linsenaufnahme. Bevorzugt tritt das Licht möglichst koaxial zum Ständer bzw. Sauger aus. Hierdurch wird es einem Benutzer vereinfacht, die Sklerallinse einzusetzen.

Der Durchmesser des Lichtleitkabels beträgt wenigstens 2 mm, vorzugsweise wenigstens 4 mm. Ein solcher Durchmesser hat sich als besonders geeignet erwiesen, um genügend Umgebungslicht aufzunehmen und mit geringer Dämpfung bis zur Austrittsstelle weiterzuleiten. Kleinere Durchmesser können zwar zu einer kompakteren Bauform führen, verringern aber die Lichtintensität am proximalen Ende. Besonders bevorzugt ist der Durchmesser wenigstens 6 mm.

Das Lichtleitkabel kann seitenleuchtend ausgebildet sein (gezielte Emission entlang der Mantelfläche, z. B. durch Mikrostreuzentren), um eine ringförmige, homogene Ausleuchtung des Saugerumfelds oder des Saugers selbst zu erzeugen, wenn dieser aus teilweise opakem Material gebildet ist. Alternativ ist es nicht-seitenleuchtend mit Endflächenemission für eine gerichtete, axiale Beleuchtung durch den Sauger. Die Auswahl hängt von der gewünschten Kontrastierung des Flüssigkeitsmeniskus und der Blendreduzierung ab.

Der Ständer ist vorzugsweise im Wesentlichen als konkav gebogener Kegelstumpf ausgebildet. Diese Geometrie verbessert Ergonomie und Sichtlinie, reduziert Eigenabschattung und erhöht die mechanische Stabilität bei gleichzeitig geringer Materialmenge. Die konkave Biegung führt das Auge intuitiv zur Einsetzposition und begünstigt die optische Führung des Lichtleiters zur Achse.

Benachbart zum distalen Ende ist gemäß einer bevorzugten Weiterbildung eine Lichteinlassöffnung vorgesehen, über die Umgebungslicht in das Lichtleitkabel eintritt. Die Öffnung kann als Fenster, Schlitz oder trichterförmige Eintrittsfläche ausgebildet sein. Sie kann optional durch eine transparente Abdeckung gegen Verschmutzung geschützt werden. Die Position nahe dem Ständerfuß erlaubt Lichteinkopplung aus breiten Raumwinkeln (Tages- und Raumlicht). Vorzugsweise ist die Lichteinlassöffnung geneigt und weist vorzugsweise einen Winkel von 60°oder weniger, vorzugsweise 45° oder weniger mit der Horizontalen auf.

In einer bevorzugten Weiterbildung verläuft das Lichtleitkabel ausgehend von der Lichteinlassöffnung zunächst gekrümmt in Richtung des distalen Endes (zur Optimierung des Einkoppelwinkels) und anschließend im Wesentlichen koaxial in Richtung des proximalen Endes. Dieser Verlauf minimiert Biegeverluste, stabilisiert die optische Achse und erleichtert die Integration des Kabels in den inneren Kanal.

In einer bevorzugten Ausführungsform weist der Ständer an seinem distalen Ende einen Saugnapf auf, mittels dessen der Ständer lösbar auf einer Arbeitsfläche befestigt werden kann. Der Saugnapf sorgt für eine stabile Fixierung der Einsetzhilfe während des Gebrauchs und verhindert ein unbeabsichtigtes Verrutschen auf glatten Oberflächen, etwa Waschbecken, Spiegelplatten oder Arbeitsflächen im Badezimmer. Diese Ausgestaltung erlaubt es, die Vorrichtung sowohl einfach zu positionieren als auch ohne Rückstände wieder zu entfernen, wodurch sich die Alltagstauglichkeit und die hygienische Handhabung verbessern. Der Saugnapf kann einstückig mit dem Ständer ausgebildet oder als separates Bauteil lösbar angebracht sein.

Der Ständer ist vorzugsweise mittels 3D-Druck herstellbar, wodurch komplexe Innenkanäle, der Aufnahmenapf und die äußere Geometrie in einem Bauteil integrierbar sind. Geeignete Werkstoffe umfassen z. B. biokompatible Photopolymere, PA12 oder PETG; die additive Fertigung erlaubt schnelle Anpassungen und kosteneffiziente Serienvarianten.

Der Ständer weist einen inneren Kanal für das Lichtleitkabel auf, in den das Lichtleitkabel formschlüssig einsetzbar ist. Insbesondere bei der Herstellung mittels 3D-Druck lässt sich der Kanal vorteilhaft einbringen. Er hat vorzugsweise einen Durchmesser derart, dass das Lichtleitkabel von einer Öffnung aus in den Kanal eingeschoben werden kann und dort durch Reibschluss gehalten wird. Der formschlüssige bzw. reibschlüssige Sitz verhindert Verdrehung/Verlust, sichert den definierten optischen Verlauf und erleichtert Montage und Austausch ohne Klebstoffe.

Die Einsetzhilfe ist in bevorzugt batterielos ausgebildet und nutzt ausschließlich Umgebungslicht. Dies reduziert Wartung, eliminiert elektrische Risiken und verbessert Reinigbarkeit und Nachhaltigkeit, ohne die Sichtunterstützung zu beeinträchtigen.

Ausführungsformen der Erfindung werden nun nachfolgend anhand der Zeichnungen beschrieben. Diese sollen die Ausführungsformen nicht notwendigerweise maßstäblich darstellen, vielmehr sind die Zeichnungen, wenn dies zur Erläuterung dienlich ist, in schematisierter und/oder leicht verzerrter Form ausgeführt. Im Hinblick auf Ergänzungen der aus den Zeichnungen unmittelbar erkennbaren Lehren wird auf den einschlägigen Stand der Technik verwiesen. Dabei ist zu berücksichtigen, dass vielfältige Modifikationen und Änderungen betreffend die Form und das Detail einer Ausführungsform vorgenommen werden können, ohne von der allgemeinen Idee der Erfindung abzuweichen. Die in der Beschreibung, in den Zeichnungen sowie in den Ansprüchen offenbarten Merkmale der Erfindung können sowohl einzeln als auch in beliebiger Kombination für die Weiterbildung der Erfindung wesentlich sein. Zudem fallen in den Rahmen der Erfindung alle Kombinationen aus zumindest zwei der in der Beschreibung, den Zeichnungen und/oder den Ansprüchen offenbarten Merkmale. Die allgemeine Idee der Erfindung ist nicht beschränkt auf die exakte Form oder das Detail der im Folgenden gezeigten und beschriebenen bevorzugten Ausführungsformen oder beschränkt auf einen Gegenstand, der eingeschränkt wäre im Vergleich zu dem in den An-sprüchen beanspruchten Gegenstand. Bei angegebenen Bemessungsbereichen sollen auch innerhalb der genannten Grenzen liegende Werte als Grenzwerte offenbart und beliebig einsetzbar und beanspruchbar sein. Der Einfachheit halber sind nachfolgend für identische oder ähnliche Teile oder Teile mit identischer oder ähnlicher Funktion gleiche Bezugszeichen verwendet.

Weitere Vorteile, Merkmale und Einzelheiten der Erfindung ergeben sich aus der nachfolgenden Beschreibung der bevorzugten Ausführungsformen sowie anhand der Zeichnungen; diese zeigen in:
- Figur 1: eine perspektivische Gesamtansicht einer Einsetzhilfe mit Ständer, Sauger, und Sklerallinse;
- Figur 2: eine Seitenansicht der Einsetzhilfe mit Darstellung des Aufnahmenapfes am proximalen Ende;
- Figur 3: eine perspektivische Draufsicht auf die Einsetzhilfe mit Lichteinlassöffnung und Lichtleiteinrichtung;
- Figur 4: Längsschnitt durch die Einsetzhilfe mit Darstellung des inneren Kanals, Lichtleitkabels, Lichteinlassöffnung, Aufnahmenapf, und weiteren Komponenten; und in
- Figur 5: eine perspektivische teilweise durchsichtige Ansicht der Einsetzhilfe.

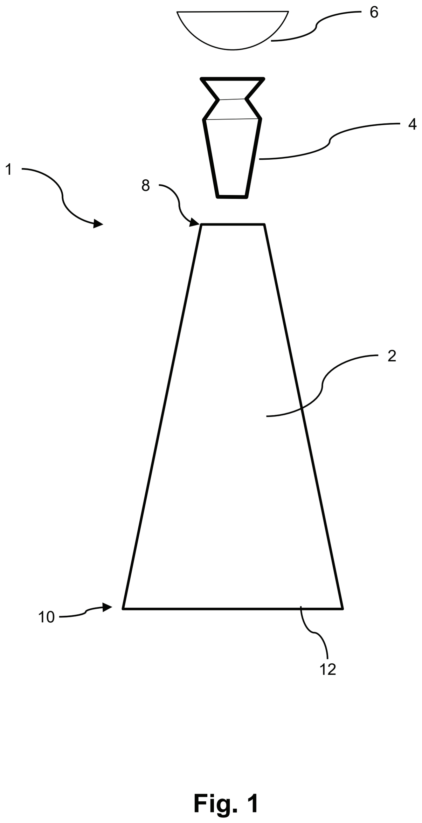

Fig. 1 zeigt eine Einsetzhilfe 1 zum Einsetzen einer Sklerallinse 6. Die Einsetzhilfe 1 umfasst einen Ständer 2, der sich zwischen einem proximalen Ende 8 und einem distalen Ende 10 erstreckt. Am distalen Ende ist ein Ständerfuß 12 vorgesehen, über den die Einsetzhilfe 1 auf einer Arbeitsfläche, beispielsweise einer glatten Ablage oder Waschbeckenplatte, aufgestellt werden kann.

Am proximalen Ende 8 ist ein Sauger 4 angeordnet, der zur Aufnahme und zum temporären Halten der Sklerallinse 6 dient. Der Sauger 4 weist eine im Wesentlichen konkave, napfförmige Aufnahmefläche auf, die so gestaltet ist, dass die Sklerallinse 6 mit ihrer konvexen Seite in Anlage gebracht werden kann. Hierdurch wird die Linse während der Vorbereitung und beim Einsetzen sicher positioniert.

Der Ständer 2 ist vorzugsweise freistehend und so gestaltet, dass die optische Achse der Sklerallinse 6 im Gebrauch im Wesentlichen vertikal ausgerichtet ist. Durch die Geometrie des Ständers 2 ergibt sich eine stabile Halteposition, welche dem Anwender ermöglicht, das Auge gezielt auf die Linse abzusenken, während beide Hände frei bleiben, um die Augenlider zu öffnen.

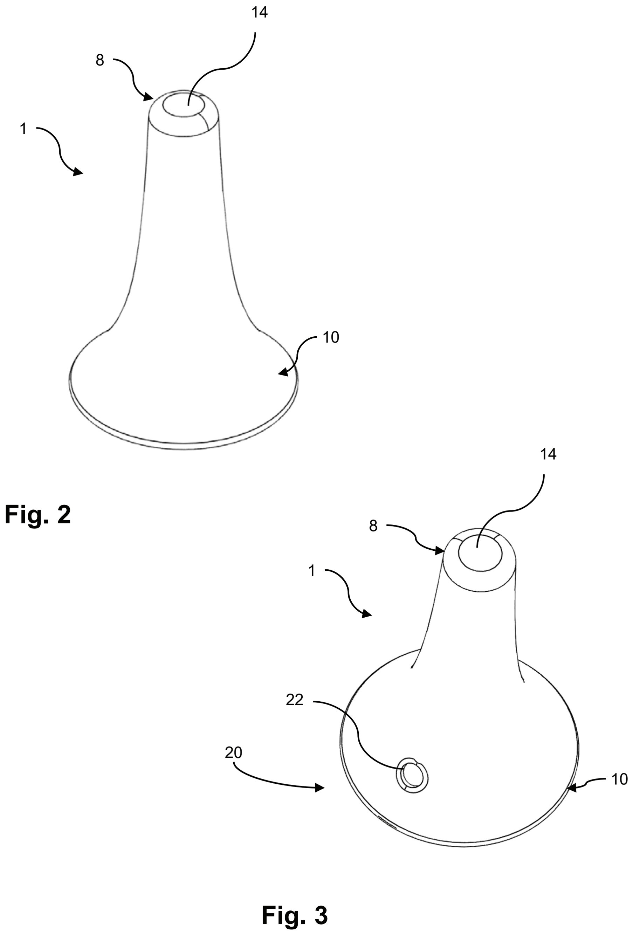

Die Bauform des Ständers 2 kann zylindrisch, konisch oder leicht konkav gebogen ausgeführt sein, um eine ergonomische Nutzung und eine gute Sicht auf die Sklerallinse 6 zu gewährleisten. In der in Fig. 1 dargestellten Ausführungsform ist der Ständer 2 nur schematisch als Kegelstumpf dargestellt. Die Figuren 2 bis 4 zeigen hingegen eine Ausführungsform, bei der der Ständer einen konkav gekrümmten Kegelstumpf bildet. Die Form kann aber im Allgemeinen frei gewählt werden und die Erfindung ist nicht auf die gezeigte Form beschränkt.

Der Ständer 2 kann aus einem transparenten, transluzenten oder opaken Material bestehen, beispielsweise aus Kunststoff. Im dargestellten Beispiel ist er als Monokörper aus einem biokompatiblen Kunststoff gebildet, wodurch eine hygienische Reinigung und eine kostengünstige Herstellung, insbesondere durch 3D-Druck oder Spritzguss, ermöglicht wird. Bevorzugt ist er aus vollständig opakem Material und äußerlich mit einer dekorativen Farbschicht versehen.

Die dargestellte Ausführungsform bildet die Grundstruktur, auf der die weiteren in den nachfolgenden Figuren gezeigten Varianten - etwa mit passiver Lichtleiteinrichtung, Aufnahmenapf oder Saugnapf - aufbauen.

Der Sauger 4 kann mit dem Ständer 2 entweder einstückig oder getrennt ausgebildet sein. Bei einer einstückigen Ausbildung werden Ständer 2 und Sauger 4 als integrales Bauteil gefertigt, beispielsweise im Spritzguss oder durch additive Fertigung. Dies ermöglicht eine besonders kompakte, stabile und dichte Konstruktion, bei der keine Füge- oder Verbindungselemente erforderlich sind. Die Reinigung wird vereinfacht, und das Risiko einer unbeabsichtigten Trennung oder Undichtigkeit zwischen Ständer 2 und Sauger 4 entfällt.

In einer alternativen Ausführung kann der Sauger 4 getrennt ausgebildet und lösbar mit dem proximalen Ende 8 des Ständers 2 verbunden sein, etwa durch Presssitz, Schnapp-, Bajonett- oder Gewindeverbindung. Diese Variante erlaubt den Austausch des Saugers 4, beispielsweise bei Verschleiß, Materialermüdung oder zur Anpassung an unterschiedliche Linsengrößen und Geometrien. Zudem kann der Sauger 4 zur Reinigung, Desinfektion oder Sterilisation separat abgenommen werden, wodurch die Hygiene und Wartungsfreundlichkeit verbessert werden. Der Sauer hat vorzugsweise eine zentrale Durchgangsöffnung, um eine bessere Beleuchtung der Sklerallinse zu ermöglichen.

Fig. 2 zeigt eine nun eine Ausführungsform der Einsetzhilfe 1, bei der der Ständer 2 eine konkave Form hat. Am proximalen Ende 8 des Ständers 2 ist ein Aufnahmenapf 14 zu erkennen. Der Aufnahmenapf 14 ist vorzugsweise napfförmig ausgebildet und dient der aufnahmegerechten Positionierung eines Saugers 4, der in den Aufnahmenapf 14 lösbar eingesetzt ist. Die lösbare Verbindung kann beispielsweise durch Presssitz realisiert werden.

Fig. 3 zeigt eine weitere Ansicht der Einsetzhilfe 1 gemäß Fig. 2. Zu sehen ist insbesondere, dass der Ständer 2 zusätzlich eine passive Lichtleiteinrichtung 20 aufweist. Konkret ist in Fig. 3 nur die Lichteinlassöffnung 22 der Lichtleiteinrichtung 20.

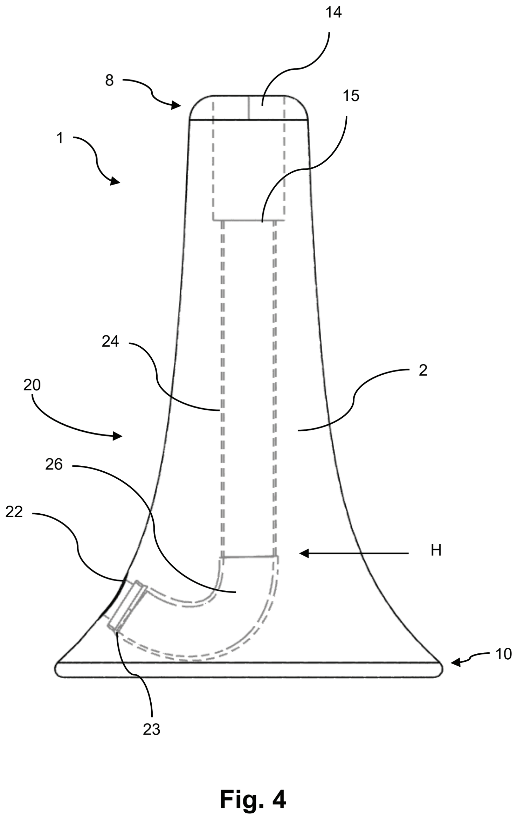

Fig. 4 zeigt einen Querschnitt der Einsetzhilfe 1 gemäß den Figuren 2 und 3. In Fig. 4 ist insbesondere die passive Lichtleiteinrichtung 20 zu sehen, welche dazu ausgebildet ist, Umgebungslicht von einer Lichteinlassöffnung 22 in den Ständer 2 einzukoppeln, entlang eines inneren Kanals 24 zu führen und im Bereich des proximalen Endes 8 - insbesondere bis zu einem Bodenabschnitt 15 des Aufnahmenapfes 14 - gezielt auszukoppeln. In der dargestellten Variante weist die Lichtleiteinrichtung 20 ein Lichtleitkabel 26 auf, das im inneren Kanal 24 eingesetzt ist.

Die Lichteinlassöffnung 22 ist am distalen Ende 10 oder benachbart dazu angeordnet und bildet eine Eintrittsfläche, über die Tages- oder Raumlicht in das Lichtleitkabel 26 eingekoppelt wird. Die Eintrittsfläche kann plan, abgeschrägt oder leicht trichterförmig ausgebildet sein, um den Einfallswinkel für ein breites Spektrum an Umgebungslicht zu optimieren. Eine geneigte Ausbildung der Eintrittsfläche relativ zur Ständeraußenkontur erleichtert die Kopplung auch bei diffusem oder seitlichem Lichteinfall. Die Lichteinlassöffnung 22 kann so ausgerichtet sein, dass sie bei typischer Aufstellung der Einsetzhilfe 1 vorzugsweise in Richtung einer helleren Umgebung (z. B. Fenster oder Leuchte) weist. Die Lichteinlassöffnung 22 kann mit einer umlaufenden Fase oder einer trichterförmigen Aufweitung versehen sein, um die Lichtkopplung zu verbessern und gleichzeitig eine mechanische Führung beim Einsetzen des Lichtleitkabels 26 zu bieten.

Von der Lichteinlassöffnung 22 ausgehend verläuft das Lichtleitkabel 26 zunächst gekrümmt in Richtung des distalen Endes 10 und anschließend im Wesentlichen koaxial zum Ständer 2 in Richtung des proximalen Endes 8. Dieser Verlauf erlaubt es, Licht aus einem großen Raumwinkel einzusammeln und zugleich die optische Achse zum Sauger 4 bzw. Aufnahmenapf 14 einzuhalten. Der Biegeradius des Lichtleitkabels 26 ist so gewählt, dass Totalreflexion über den größten Teil des Wegs erhalten bleibt, wodurch Dämpfungsverluste minimiert werden. Der innere Kanal 24 führt das Lichtleitkabel 26 passgenau und verhindert Knicke; lokale Führungsabsätze oder Schulterungen können die Position zusätzlich fixieren.

Das Lichtleitkabel 26 besteht bevorzugt aus einem transparenten Polymer, z. B. PMMA oder Polycarbonat, alternativ aus Glasfasermaterial. Im vorliegenden Ausführungsbeispiel hat das Lichtleitkabel 26 einen Durchmesser von 6 mm. Der Querschnitt kann rund ausgeführt sein; polygonale oder leicht elliptische Querschnitte sind möglich, sofern die Kopplungs- und Führungseigenschaften erhalten bleiben. Die Stirnflächen am Ein- und Austritt sind vorzugsweise poliert, um Reflexionsverluste zu reduzieren.

Das Lichtleitkabel 26 kann nicht-seitenleuchtend oder seitenleuchtend ausgeführt sein: In der nicht-seitenleuchtenden Ausführung wird das Licht primär axial geleitet und am proximalen Ende aus dem Lichtleitkabel 26 auskoppelt. Diese Variante unterstützt eine gerichtete Beleuchtung unmittelbar durch den Sauger 4 bzw. in den Aufnahmenapf 14 hinein. In der seitenleuchtenden Ausführung treten definierte Anteile des Lichts über die Mantelfläche aus. Dies erzeugt im Bereich des Aufnahmenapfes 14 eine diffuse Hintergrundaufhellung, welche Meniskus-Kanten der Sklerallinse 6 gut sichtbar macht. Eine seitenleuchtende Charakteristik kann durch gezielte Mikro-Rauhungen oder streuende Einschlüsse entlang eines Abschnitts des Lichtleitkabels 26 erzielt werden. Beide Varianten können auch kombiniert werden, etwa indem das Kabel überwiegend nicht-seitenleuchtend ausgebildet ist und nur nahe dem Bodenabschnitt 15 eine definierte Streuzone aufweist, die eine weiche, blendfreie Ausleuchtung des Linsenaufnahmeraums bewirkt.

Das Austrittsende des Lichtleitkabels 26 ist vorzugsweise bis zum Bodenabschnitt 15 des Aufnahmenapfes 14 geführt. Dort tritt das Licht entweder punkt- bzw. flächenförmig aus. Optional kann am Austrittsende ein Diffusorelement oder eine mikrostrukturierte Fläche vorgesehen sein, um Hot-Spots zu vermeiden und eine gleichmäßige Helligkeitsverteilung zu erzielen. Dadurch werden Konturen der Sklerallinse 6 sowie der Flüssigkeitsmeniskus zuverlässig erkennbar, was das blasenfreie Einsetzen unterstützt.

Alternativ kann das Lichtleitkabel auch kürzer ausgeführt sein, und etwa auf Höhe des Pfeils H in Fig. 4 enden, als sich im Wesentlichen lediglich über den gekrümmten Abschnitt des Kanals 24 erstrecken, nicht aber über den geraden.

Das Lichtleitkabel 26 ist lösbar oder dauerhaft in den inneren Kanal 24 einsetzbar. Bei lösbarer Ausführung gewährleistet die formschlüssige Passung einen sicheren Sitz und erlaubt den Austausch im Servicefall. Alternativ kann eine stoffschlüssige Fixierung (z. B. Kleben) gewählt werden. Die im Ständer 2 vollständig gekapselte Führung ohne offene Hinterschneidungen begünstigt die Reinigbarkeit und verhindert Flüssigkeitsretention. Alle dem Flüssigkeitskontakt ausgesetzten Flächen sind medienbeständig und glatt ausgebildet, um eine hygienische Aufbereitung (Spülen, Desinfektion) zu erleichtern.

Der Ständer 2 kann im 3D-Druck oder Spritzguss hergestellt werden. Beim 3D-Druck lassen sich innere Kanäle 24 mit definierten Radien und Aufnahmeschultern für das Lichtleitkabel 26 in einem Schritt erzeugen. Beim Spritzguss kann der innere Kanal 24 mittels Kernzug oder lösbarem Einsatz ausgebildet werden. Das Lichtleitkabel 26 kann anschließend eingedrückt, eingeschnappt oder eingeschoben werden. In dem vorliegenden Ausführungsbeispiel ist an der Lichteinlassöffnung 22 eine Ringschulter 23 als Anschlag vorgesehen. Das Lichtleitkabel 26 wird dann bei der Montage ausgehend von dem Aufnahmenapf 14 durch eine Öffnung in dem Bodenabschnitt 15 in den Kanal 24 eingeschoben, bis es mit der Ringschulter 23 in Kontakt kommt.

Die beschriebene Ausführung der Lichtleiteinrichtung 20 sorgt dafür, dass ohne aktive Lichtquelle eine für das Einsetzen ausreichende Beleuchtungsstärke im Bereich der Sklerallinse 6 erzielt wird. Die gerichtete oder diffuse Auskopplung nahe dem Bodenabschnitt 15 verbessert die Sichtbarkeit des Flüssigkeitsmeniskus und erleichtert die Zentrierung der Linse relativ zum Auge. Gleichzeitig bleibt die Einsetzhilfe 1 batterielos, robust und wartungsarm, wodurch Nachhaltigkeit und Alltagstauglichkeit erhöht werden.

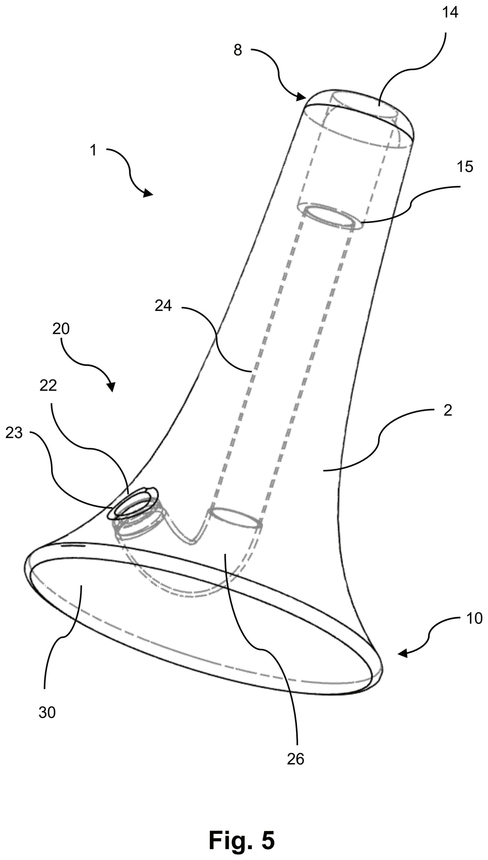

Fig. 5 zeigt eine weitere Ansicht der Einsetzhilfe 1, bei der der Ständer 2 an seinem distalen Ende 10 mit einem Saugnapf 30 versehen ist. Der Saugnapf 30 dient der lösbaren Befestigung der Einsetzhilfe 1 auf einer Arbeitsfläche, beispielsweise auf einer glatten Ablage, einem Waschbeckenrand oder einer Tischplatte. Er sorgt für eine stabile und kippsichere Fixierung der Einsetzhilfe 1 während des Gebrauchs und verhindert ein unbeabsichtigtes Verrutschen oder Umkippen bei der Handhabung der Sklerallinse 6.

Der Saugnapf 30 kann integraler Bestandteil des Ständers 2 oder als separates Bauteil ausgebildet sein, das mit dem distalen Ende 10 über einen Steck-, Klemm- oder Schraubanschluss verbunden ist. In einer besonders einfachen Ausführung ist der Saugnapf 30 einstückig mit dem Ständer 2 gefertigt, beispielsweise im Spritzguss oder 3D-Druck. Diese integrale Bauweise gewährleistet eine dauerhaft dichte Verbindung ohne separate Dicht- oder Verbindungselemente und erleichtert die Reinigung.

Alternativ kann der Saugnapf 30 lösbar an dem Ständer 2 angebracht sein, sodass er bei Bedarf ausgetauscht oder separat gereinigt werden kann. Dies ist insbesondere vorteilhaft, wenn die Vorrichtung regelmäßig desinfiziert oder sterilisiert wird, oder wenn ein Saugnapf aufgrund von Alterung oder Materialermüdung ersetzt werden soll.

Alternativ oder zusätzlich kann der Ständerfuß 12 eine strukturierte oder gummierte Oberfläche aufweisen, um die Haftung auf glatten Flächen zu verbessern und ein sicheres Handling beim Aufstellen der Einsetzhilfe 1 zu gewährleisten.

## Patentansprüche

1. Einsetzhilfe (1) zum Einsetzen von Sklerallinsen (6), mit
einem Ständer (2) aufweisend ein proximales Ende (8) und ein distales Ende (10), wobei der Ständer (2) an dem distalen Ende (10) einen Ständerfuss (12) zum Aufstellen auf einer Arbeitsfläche aufweist, und
einem Sauger (4) zum Aufnehmen der Sklerallinse (6), wobei der Sauger (6) an dem proximalen Ende (8) des Ständers (2) angeordnet ist, wobei
der Ständer (2) eine passive Lichtleiteinrichtung (20) zum Leiten von Umgebungslicht durch den Ständer (2) bis zum proximalen Ende (8) des Ständers (2) aufweist, zum Beleuchten der Sklerallinse (6) durch den Sauger (4) hindurch.

2. Einsetzhilfe nach Anspruch 1, aufweisend einen Aufnahmenapf (14) an dem proximalen Ende (8), in den der Sauger (6) lösbar einsetzbar ist.

3. Einsetzhilfe nach einem der vorstehenden Ansprüche, wobei die Lichtleiteinrichtung (20) ein Lichtleitkabel (26) aufweist, welches sich von einer Außenseite des Ständers (2) bis zum proximalen Ende (8) des Ständers (2) erstreckt.

4. Einsetzhilfe nach den Ansprüchen 2 und 3, wobei sich das Lichtleitkabel (26) bis zu einem Bodenabschnitt (15) des Aufnahmenapfes (14) erstreckt.

5. Einsetzhilfe nach Anspruch 3 oder 4, wobei das Lichtleitkabel (26) einen Durchmesser von wenigstens 2 mm, vorzugsweise wenigstens 4 mm aufweist.

6. Einsetzhilfe nach einem der Ansprüche 3 bis 5, wobei das Lichtleitkabel (26) seitenleuchtend oder nicht-seitenleuchtend ist.

7. Einsetzhilfe nach einem der vorstehenden Ansprüche, wobei der Ständer (2) im Wesentlichen eine Form eines konkav gebogenen Kegelstumpfs hat.

8. Einsetzhilfe nach Anspruch 3 und 7, wobei der Ständer (2) benachbart zum distalen Ende (10) eine Lichteinlassöffnung (22) hat, über die Licht in das Lichtleitkabel (26) eintreten kann.

9. Einsetzhilfe nach Anspruch 8, wobei sich das Lichtleitkabel (26) ausgehend von der Lichteinlassöffnung (22) zunächst gekrümmt in Richtung des distalen Endes (10) und dann im Wesentlichen koaxial in Richtung des proximalen Endes (8) verläuft.

10. Einsetzhilfe nach einem der vorstehenden Ansprüche, wobei der Ständer (2) am distalen Ende einen Saugnapf (30) aufweist.

11. Einsetzhilfe nach einem der vorstehenden Ansprüche, wobei der Ständer (2) mittel 3D-Druck hergestellt ist.

12. Einsetzhilfe nach Anspruch 3, wobei der Ständer (2) einen inneren Kanal (24) für das Leichtleitkabel (26) aufweist, in den das Leichtleitkabel (26) reib- und/oder formschlüssig gehalten ist.

13. Einsetzhilfe nach einem der vorstehenden Ansprüche, wobei die Einsetzhilfe batterielos ausgebildet ist.
